# NEW EUROPEAN PATENT SPECIFICATION

(11) **EP 1 652 533 B2**
(45) Date of publication and mention of the opposition decision: **19.11.2014**
(45) Mention of the grant of the patent: 16.11.2011
(21) Application number: 04771640.2
(22) Date of filing: 06.08.2004
(51) Int. Cl.: A61K 45/00, A61K 31/5377, A61K 49/00, A61K 31/195, A61P 9/00

(54) **HEART-SLOWING DRUG CONTAINING SHORT-ACTING BETA-BLOCKER AS THE ACTIVE INGREDIENT**
HERZVERLANGSAMENDER ARZNEISTOFF MIT BETA-BLOCKERN MIT KURZZEITWIRKUNG ALS WIRKSTOFF
MEDICAMENT DE RALENTISSEMENT DE BATTEMENT DE COEUR CONTENANT UN BETA-BLOQUANT A ACTION BREVE EN TANT QUE PRINCIPE ACTIF

(30) Priority: 08.08.2003 JP 2003290301
(43) Date of publication of application: 03.05.2006
(73) Proprietor: ONO PHARMACEUTICAL CO., LTD., Osaka-shi, Osaka 541-8526 (JP)
(72) Inventor: HIRANO, Masaharu, Setagaya-ku, Tokyo 158-0083 (JP); NAKAO, Kenzo, Osaka-shi, Osaka 541-0056 (JP); MIZUSHIMA, Ken,, Osaka-shi, Osaka 541-0056 (JP); AKISADA, Morito,, Tokyo 101-0041 (JP); AZUMA, Takahiro, Tokyo 101-0041 (JP)
(74) Representative: Duckworth, Timothy John
(86) International application number: PCT/JP2004/011672
(87) International publication number: WO 2005/014042

(56) References cited:
- EP-A1- 1 652 533
- JP-A- 3 072 475
- JP-A- 57 502 123
- US-A- 5 856 342
- US-A1- 2003 064 097
- IGUCHI S ET AL: "DEVELOPMENT OF A HIGHLY CARDIOSELECTIVE ULTRASHORT-ACTING BETA-BLOCKER ONO-1101" CHEMICAL AND PHARMACEUTICAL BULLETIN, vol. 40, no. 6, 1992, pages 1462-1469, XP002546967
- AHMET I ET AL: "The effects of a new ultra-short-acting beta-adrenergic blocker, ONO-1101, on cardiac function during and after cardiopulmonary bypass" SURGERY TODAY, vol. 29, no. 3, 1999, pages 248-254, XP002546968
- KITAMURA A ET AL: "Efficacy of an ultrashort-acting beta-adrenoceptor blocker (ONO-1101) in attenuating cardiovascular responses to endotracheal intubation" EUROPEAN JOURNAL OF CLINICAL PHARMACOLOGY, vol. 51, no. 6, 1997, pages 467-471, XP002546969
- HAYASHI Y ET AL: "Bolus Administration of Landiolol, a Short Acting and Selective beta1 Blocker, To Treat Intraoperative Tachycardia. A Dose-Dependent Study." ANESTHESIOLOGY ABSTRACTS OF SCIENTIFIC PAPERS ANNUAL MEETING, 2003, page A 193, XP002546970 & 2003 ANNUAL MEETING OF THE AMERICAN SOCIETY OF ANESTHESIOLOGISTS; SAN FRANCISCO, CA, USA; OCTOBER 11-15, 2003
- DATABASE MEDLINE [Online] US NATIONAL LIBRARY OF MEDICINE; 1986, ANGARAN D M et al: "Esmolol hydrochloride: an ultrashort-acting, beta-adrenergic blocking agent." XP002546971 Database accession no. NLM2871961
- HONG C ET AL: "ECG-gated reconstructed multi-detector row CT coronary angiography: Effect of varying trigger delay on image quality" RADIOLOGY, vol. 220, no. 3, 2001, pages 712-717, XP002546972
- DATABASE MEDLINE [Online] US NATIONAL LIBRARY OF MEDICINE; 1994, SALBAS K et al: "Interaction between beta-blockers and contrast media during intracoronary administration in rabbit heart." XP002546973 Database accession no. NLM8305278
- SCHROEDER S ET AL: "Influence of heart rate on vessel visibility in noninvasive coronary angiography using new multislice computed tomography. Experience in 94 patients" CLINICAL IMAGING, vol. 26, no. 2, 2002, pages 106-111, XP002546974
- ROPERS D ET AL: "Detection of coronary artery stenoses with thin-slice multi-detector row spiral computed tomography and multiplanar reconstruction." CIRCULATION, vol. 107, no. 5, 11 February 2003 (2003-02-11), pages 664-666, XP002546975
- LEBER A.W. ET AL.: 'Non-invasive intravenous coronary angiography using electron beam tomography and multislice computed tomography' HEART vol. 89, no. 6, June 2003, pages 633 - 639, XP002985389
- SATO Y. ET AL.: 'Kyusei kanshokogun to multislice CT' THE JAPANESE JOURNAL OF ACUTE MEDICINE, 2003 NEN 6 GATSU vol. 27, 2003, pages 719 - 725, XP002985390
- NIEMAN K. ET AL.: 'Reliable noninvasive coronary angiography with fast submillimeter multislice spiral computed tomography' CIRCULATION vol. 106, no. 16, 2002, pages 2051 - 2054, XP002985391
- T. C. GERBER ET AL.: 'Image Quality in a Standardized Algorithm for Minimally Invasive Coronary Angiography with Multislice Spiral Computed Tomography' JOURNAL OF COMPUTER ASSISTED TOMOGRAPHY vol. 27, no. 1, 2003, pages 62 - 69
- OGATA ET AL. CORRESPONDENCE vol. 50, no. 7, August 2003, page 753
- Konishi R. et al.. Masui, 2003, May; 52(5): 515-518 (engl. translation D3a)
- FUSTER ET AL.: 'ACC/AHA/ESC Guidelines for the Management of Patients With Atrial Fibrillation: Executive Summary' JOURNAL OF THE AMERICAN COLLEGE OF CARDIOLOGY vol. 38, no. 4, 2001, pages 1231 - 1265
- A.W. LEBER ET AL.: 'Non-invasive intravenous coronary angiography using electron beam tomography andmultislice computed tomography' CARDIOVASCULAR MEDICINE vol. 89, 2003, pages 633 - 639
- Declaration Mr. Tatsuaki Okamura
- Kato K. et al., 1997, Jpn.J.Clin.Pharmacol.Ther., 19,4873 (53)-4901(81) (engl. translation)
- Yoshiya I. et al., 2000, J. Ciin.Ther. & Med., 16(10, 1557 - 1577 (engl. translation), cited in D3
- Yoshiya I. et al., 2002, Jpn.J.Clin Pharmacol.Ther., 18,9, 1049 (37) - 1076 (64)(engl. translation)
- Press Release Ono Pharmaceuticals CO., Ltd. July 23, 2002
- Press Release Ono Pharmaceuticals Co., Ltd. July 1, 2011
- Product Description "Intravenous Corebeta 12.5 g", Sept. 2011
- JP2003-290301, filing date 8.8.2003 inc. translation (P1a)

## Description

### Technical Field

The present invention relates to an agent which slows down the heart rate, comprising a short-acting β-blocker as active ingredient.

More specifically, the present invention relates to an agent which slows down the heart rate in diagnostic imaging, comprising the short-acting β-blocker landiolol hydrochloride as active ingredient

### Background Art

It is said that, two inspections are required to diagonize ischemic heart diseases conclusively, i.e. "a stress myocardial scintigram inspection" for the purpose of detecting an ischemic site of a coronary artery and "a heart catheter inspection (coronary angiography inspection)" for detecting a stenosed site of a coronary artery. Since among them, the heart catheter inspection costs too much and it can be performed only by professional doctors and it includes a risk of death due to complications. Therefore, less expensive, easily performable in the out-patient clinic and non-invasive inspections have been hoped for.

In such situations, coronary angiography which uses multi-slice helical computed tomography (MSCT), which is also called multi-detector helical computed tomography (MDCT), it is abbreviated as MSCT hereafter, fulfills these conditions and it is remarked as an inspection method which replaces the heart catheter inspection.

With the images given from a conventional CT, only a diagnosis by planar images vertical to the body axis can be performed, while MSCT can photograph more planar images in a short time because of photographing plural planar images at the same time, and thereby it is possible to reconstitute three-dimensional images. Therefore, it is possible to detect the stenosis site of a coronary artery based on the images by constituting images of a coronary artery which runs in three dimensions and complicatedly. However, since MSCT has low temporal resolution, it is subject to the heart rate and respirations.

Therefore, at present, in the medical field, sharp images are obtained by slowing down the heart rate as a result of administration of oral β-blockers (see Journal of Japan Radiological Society, 1993, Vol,53, No.9, 1033-1039 and The Japanese Journal of Acute Medicine, 2003, Vol.27, 719-725).

However, existing oral β-blockers have some troubles; i.e. (1) it takes some time to show the drug effect and to disappear the drug effect, and therefore it takes long time from administration to inspection, and observation after inspection, (2) if there is individual distinction in the time until the effect of the drug arises and it takes some time to onset of the drug effect, the inspection will be troublesome, (3) it is hard to adjust the dose because of bad controlability in the heart rate, (4) there is fear of postoperative safety (e.g. lowering of blood pressure *etc.* because of continuation of β-blocking effect), *etc.*

Since it takes 10 to 15 minutes to adjust the heart rate in MSCT diagnosis, the patients who will have an inspection cannot move safely after inspection if the slowing down of the heart rate continues too long. The situation like this is not necessarily preferable for the patients.

Therefore, a drug which excellently adjusts various kinds of diagnostic imaging has been hoped for the purpose of improvement of angiography such as MSCT, improvement of safety for patients, shortening of the inspection time, *etc.*

On the other hand, (-)-[(S)-2,2-dimethyl-1,3-dioxolan-4-yl]methyl-3-[4-[(S)-2-hydroxy-3-(2-morpholinocarbonylamino)ethylamino]propoxy]phenylpropionic acid hydrochloride (CAS registry number: 144481-98-1; called landiolol hydrochloride hereafter) is used in emergency treatment of intraoperative and tachyarrhythmia (fibrillation, atrial flutter, sinus tachycardia) as a short-acting β1-blocker (e.g. see the gazette of JP 3-72475).

Landiolol hydrochloride is quickly decomposed by esterases in blood and a liver to inactive compounds and it has an extremely short half life in blood of approximately 4 minutes, compared with existing β-blockers. The selectivity on β₁ (β₁/β₂) is approximately 250 and so it has a high selectivity on a heart, and it is to be estimated it has little influence on airway system.

### Disclosure of the Invention

As a result of energetic investigation in view of the above situations, the present inventors have found out that a short-acting β-blocker, surprisingly, shows an excellent effect as an agent which slows down the heart rate in diagnostic imaging including MSCT as active ingredient.

A short-acting β-blocker like this is especially useful in the short-term diagnosis because it is easy to control the heart rate in the use of infused intravenous administration. Compared with the existing pharmaceutical agents, for example, landiolol hydrochloride has a very short period of a half life (3 to 5 minutes) in the blood, therefore, it is possible to adjust the heart rate by changing the dose and the method of administration.

Hereby use of a short-acting β-blocker having convenience in controlability avoids the problem of existing β-blockers, improves the ability of angiography by MSCT, and consequently avoids adverse effects (staggering, vertigo, etc.) after angiography of the patients.

The present invention is:
1. A compound which is landiolol hydrochloride for use in slowing the heart rate of a patient during diagnostic imaging using multi-slice helical computed tomography (MSCT), wherein the compound is administered in a swift intravenous administration of the compound at 0.063 to 0.125 mg/kg/minute for 30 seconds to 3 minutes.
2. The compound according to claim 1 for use according to above 1, wherein the landiolol hydrochloride is provided to the patient in said swift intravenous administration additionally followed by administration of the compound at 0.01 to 0.08 mg/kg/minute for 5 to 20 minutes.
3. A compound which is landiolol hydrochloride for use in slowing the heart rate of a patient during diagnostic imaging using multi-slice helical computed tomography (MSCT), wherein the compound is administered in a swift intravenous administration of from 0.063 to 0.125 mg/kg/minute of from 30 seconds to 3 minutes followed by a sustained intravenous administration of from 0.01 to 0.08 mg/kg/minute of from 5 to 20 minutes.
4. The compound according to claim 1 for use according to above 1, 2 or 3, wherein the diagnostic imaging is diagnostic imaging for heart diseases.
5. The compound according to claim 1 for use according to above 1, 2, 3 or 4, wherein a contrast medium is also administered.
6. The compound according to claim 1 for use according to above 5, wherein the contrast medium is chosen from an iodinated contrast medium, a borated contrast medium, a xenon contrast medium, a barium contrast medium, a ferrous contrast medium and a gadolinium contrast medium.

The diseases in which a short-acting β-blocker is used are for example, visceral diseases such as heart diseases.

Heart diseases include, ischemic heart diseases (e.g. angina pectosis, cardiac infarction, *etc*.), arrhythmia, *etc*. and ischemic heart diseases are preferable.

The short-acting β-blocker used according to the invention is landiolol hydrochloride.

In the present invention, the short-acting β-blocker may be used alone or may be used with e.g. muscle relaxant agents, antianxiety agents, anesthetic adjuncts, *etc.*

Muscle relaxant agents used in the present invention to be used in combination with the short-acting β-blocker include, e.g. botulinus toxin type A, papaverine hydrochloride, dantrium, dantrolene sodium, vecuronium bromide, pancuronium bromide, suxamethonium chloride, *etc.*

Antianxiety agents used in the present invention to be used in combination with the short-acting β-blocker include, e.g. diazepam, oxazolam, flutazolam, alprazolam, ethyl loflazepate, tofisopam, etizolam, bromazepam, clotiazepam, lorazepam, *etc.*

Anesthetic adjuncts used in the present invention to be used in combination with the short-acting β-blocker include, e.g. pethidine, fentanyl, dromoran, *etc*.

In the present invention, the heart rate may be controlled by adjusting the dose and the method for administration of the short-acting β-blocker minutely. Thereby, necessary and sufficient effect will be drawn in the diagnostic imaging.

Specifically, for example, the dose of landiolal hydrochloride is preferably, (i) after performing a swift intravenous administration of a high dose, and then (ii) performing a sustained intravenous administration of a low dose.

For example, the high dose for the bolus (swift intravenous) administration for a short period in the process (i) is, a necessary dose in order to acquire steady blood in the introduction of landiolol hydrochloride. Specifically, 0.063 to 0.125 mg/minute per 1 kg of the patient. The short period in the process (i) is the time in order to acquire the steady blood concentration in the introduction of landiolol hydrochloride. Particularly, 30 seconds to 3 minutes, more preferably 30 seconds to 2 minutes. Most preferable is 1 minute.

The swift intravenous administration in the process (i) is to administer the above dose for a short period, e.g. an administration using an infusion pump, a volumetric pump, *etc*. or manual administration.

The low dose in the process (ii) for the infusion (sustained intravenous) administration is, a sufficient dose for slowing down the steady heart rate of the patient who goes through a CT inspection. Specifically, approximately 0.01 to 0.08 mg/minute per 1 kg of the patient. More preferably, approximately 0.02 to 0.04 mg/minute per 1 kg of the patient. The long period in the process (ii) is, a sufficient time for acquiring the lowering of the steady heart rate of the patient who goes through a CT inspection. Preferably, approximately 5 to 20 minutes, and more preferably, approximately 10 minutes.

The sustained intravenous administration in the process (ii) is to administer the above dose for a long period, e.g. an administration using an infusion pump, a volumetric pump, *etc*. or manual administration.

In the present invention, the purpose of swift intravenous administration of a high dose in the process (i) is to upper the blood concentration of landiolol hydrochloride immediately, while the purpose of intravenous administration of a low dose in the process (ii) is to maintain the blood concentration of landiolol hydrochloride, whose half life is short.

"Swift intravenous administration" means administration of a high dose above for a short period and it has the same meaning as bolus administration. "Sustained intravenous administration" means administration of a low dose above for a long period and it has the same meaning as infusion administration.

The ratio of the "high dose in the process (i)" and the "low dose in the process (ii)" is preferably approximately 2:1 to 5:1, provided that the process (ii) is done followed by the process (i), and more preferably approximately 2:1 to 4:1, and particularly preferably approximately 3:1.

The doses of the second process (i) and (ii) to be administered followed by the first process (i) and (ii) have higher amount than the first process (i) and (ii) respectively.

When the combination administration of the process (i) and (ii) is done once, (1) (i) approximately 0.063 mg/kg/minute → (ii) approximately 0.02 mg/kg/minute or (2) (i) approximately 0.125 mg/kg/minute → (ii) approximately 0.04 mg/kg/minutes preferable and particularly (1) is more preferable.

When the combination administration of the process (i) and (ii) is done twice,(i) approximately 0.063 mg/kg/minute → (ii) approximately 0.02 mg/kg/minute → (i) approximately 0.125 mg/kg/minute → (ii) approximately 0.04 mg/kg/minute is preferable.

Other than those above, landiolol hydrochloride may be administered by easier method. For example, it is also effective; one ampoule (50 mg) of a freeze-dried formulation of landiolol hydrochloride (brand name: ONOACT 50 (manufactured by Ono Pharmaceutical Co., Ltd.) is dissolved in saline (20 ml) and 1 ml of the resulting solution is administered 2-3 times approximately every 20 minutes (i.e. 2.5 mg), and while confirming its effect, the solution is equipped in a syringe pump to adjust the dose to be administered by intravenous injection.

Particularly, in the severe patients, it is safer to adjust from the low dose under examining the change of the heart rate.

The heart rate to be adjusted by the compounds used in the present invention is preferably, the heart rate wherein a sharp image is given in the diagnostic imaging and no excess bradycardia is given; i.e. particularly 45 to 65 beats per minute, more preferably 50 to 60 beats per minute.

In the present invention, MSCT is used for diagnostic imaging.

In the present invention, the target organs for angiography include, for example, a heart, coronary arteries, kidneys, a liver, a uterus, a stomach, intestines, lungs and thoracic aortas, *etc.* and are not limited to them. More preferably are a heart or coronary arteries, and coronary arteries are most preferable.

The short-acting β-blocker in the present invention is optionally used with contrast media.

Contrast media are not limited in particular, but for example, iodinated contrast media (e.g. amidotrizoic acid, ioxaglic acid, ioxilan, iotalamic acid, meglumine iotroxate, iotrolan, iopanoic acid, iopamidol, iopromide, iohexol, iomeprol, sodium iopodate, metrizoic acid, iodamide, iodoxamic acid, iodine addition products of the ethyl esters of the fatty acid obtained from poppyseed oil, *etc*.), xenon contrast media (e.g. xenon gas, xenon injection solution, *etc*.), barium contrast media (e.g. barium sulfate *etc*.), ferreous contrast media (e.g. ferumoxides, iron ammonium citrate, *etc*.), gadolinium contrast media (e.g. meglumine gadopentetate, gadoteridol, *etc*.),

Radioactive isotopes used in radiocontrast angiography include, e.g. hydrogen, carbon, nitrogen, oxygen, fluorine, technetium, thallium, iodine, *etc*. Particularly, thallium chloride (201TlCl), meta-iodo-benzylguanidine (123I-MIBG), carbon dioxide, carbon monooxide, oxygen, fluoroglucose, hydrogen cyanide, *etc*.

In PET (positron-emission tomography), ultra short-lived radionuclides (11C, 13N, 150, 18F, *etc*.) may also be preferably used.

Tomographic MSCT equipments in the present invention, for example, include the followings; Aquilion 16, Aquilion 8. Aquilion 4, Aquilion/multi, Asteion/dual (above are manufactured by Toshiba), IDT16 (manufactured by Philips Medical Systems), Sensation Cardiac, Sensation 16, Sensation 10, Emotion 6, Volume class/sensation 4, Emotion Dio (manufactured by Siemens AG), ROBUSTO series (manufactured by Hitachi Medical Corporation), Mx8000 (manufactured by Philips), LightSpeed Ultra 16, LightSpeed Ultra, LightSpeed Plus/Qx/i series, HiSpeed QX/i, HiSpeed NX/i series, ProSpeed FII (manufactured by GE Yokogawa Medical System), *etc.*

As the numbers of the detector rows in the tomographic equipment, 4-slices, 8-slices, 16-slices, 32-slices and 64-slices are all preferable, and as the slices increase in number, the image becomes sharper, and as the time for inspection is shortened, 16-slices, 32-slices and 64-slices are more preferable, therefore.

In the present invention, the β-blocker antagonizes β₁, β₂ and β₃ receptors, acting on the heart specifically, on β₁ receptor.

Landiolol hydrochloride used in the present invention is described in JP 2,004,651(B) and JP 3,302,647(B) and its chemical name is (-)-[(S)-2,2-dimethyl-1,3-dioxolan-4-yl]methyl-3-[4-[(S)-2-hydroxy-3-(2-morpholinocarbonylamino)ethylamino]propoxy]phenylpropionic acid hydrochloride.

Landiolol hydrochloride is used in various forms of pharmaceutical composition according to known methods, e.g. JP 2,004,651(B) or JP 3,302647(B). For example, any formulation with which intravenous administration is applied is preferable, and particularly liquid formulation and freeze-dried formulation which is dissolved in a solubilizing agent before use. In such formulations, approximately 1 to 1,000 mg of landiolol hydrochloride is preferably contained, more preferably approximately 10 to 100 mg and much more preferably approximately 50 mg.

In the pharmaceutical composition, for example, additives are selected from excipients, binding agents, moistening agents, stabilizers, *etc*.

Administration of the short-acting β-blockers used in the present invention is preferably injection, which is applicable to the purpose of adjusting the heart rate appropriately.

Injective formulation is preferably, liquid formulation or freeze-dried formulation which will be dissolved in an solubilizing agent before use.

Liquid agents are used by dissolving, suspending or emulsifying one active substance or more in solubilizing agents.

Solubilizing agents include, for example, distilled water for injection, vegetable oil, propylene glycol, polyethylene glycol, alcohols such as ethanol, *etc*, and a combination thereof.

The present formulation may further include, e.g. a stabilizing agent (e.g. sodium citrate, sodium edetate), a solubilizing agent (e.g. glutamic acid, aspartic acid, polysorbate 80 (registered trademark), *etc*.), a suspending agent (e*.*g. surfactants such as stearyl triethanolamine, sodium lauryl sulfate, lauryl aminopropionic acid, lecithin, benzalkonium chloride, benzethonium chloride, glyceryl monostearate, polyethoxylated hydrogenated castor oil, polysorbate, *etc*., multiple alcohols such as glycerine, macrogol, *etc.,* sugars such as sorbitol, mannitol, sucrose, *etc*., celluloses such as methyl cellulose, carboxymethyl cellulose, hydroxypropylmethyl cellulose, *etc.,* hydrophilic macromolecules such as polyvinyl alcohol, polyvinylpyrrolidone, carboxyvinyl polymer, sodium carboxymethyl cellulose, methyl cellulose, hydroxymethyl cellulose, hydroxyethylcellulose, hydroxypropyl cellulose, *etc*., chondroitin sulfate, *etc.*)*,* an emulsifying agent (e.g. glycerine ester, saponin (sophora saponin, quillai extract, soybean saponin, *etc.*)*,* sucrose fatty acid ester (e.g. sucrose ester, *etc*). lecithin (e.g. vegetable lecithin, yolk lecithin, *etc.*)*,* a soothing agent (e.g. benzyl alcohol, chlorobutanol, propyleneglycol, ethyl aminobenzoate, lidocaine), a buffering agent (e.g. phosphates (sodium hydrogenphosphate, sodium dihydrogen phosphate, *etc*.), boric acid, borax, acetate (e.g. sodium acetate *etc*.), a carbonate (e.g. sodium carbonate, calcium carbonate, potassium carbonate, *etc*.), citric acid, sodium L-glutamate, *etc*.), a pH adjusting agent (e.g. sodium hydroxide, potassium hydroxide, trisodium phosphate, disodium hydrogen phosphate, hydrochloric acid, nitric acid, citric acid, boric acid, acetic acid, *etc.*)*,* a preserving agent (e.g. paraoxybenzoates such as propyl paraoxybenzoate, butyl paraoxybenzoate, parabens such as methylparaben, ethylparaben, propylparaben, butylparaben, *etc*., inverted soap such as benzalkonium chloride, benzethonium chloride, chlorhexidine gluconate, cetylpyridium chloride, *etc*., alcohol derivatives such as chlorobutanol, benzylalcohol, phenethylalcohol, *etc*., organic acids and salts thereof such as sodium dehydroacetate, sorbic acid, sodium sorbate, phenols such as parachloromethoxyphenol, parachloromethacresol, *etc*.), a tonicity agent (e.g. glucose, D-sorbitol, sodium chloride, glycerine, D-mannitol, potassium chloride, concentrated glycerine, propylene glycol, sucrose, *etc*.), *etc.* They are sterilized in the final process or manufactured by aseptic manipulation. Otherwise, sterile solid compositions, for example, freeze-dried products are manufactured and they may be sterilized or dissolved in sterile purified water or other solvents before use.

### Brief Description of the Drawing

Fig. 1 is one example of graphic image of angiography when landiolol hydrochloride is administered.
Fig. 2 is one example of graphic image of angiography when landiolol hydrochloride is not administered.

### Best Mode for Carrying out the Invention

The present invention is illustrated by the following examples but the present invention is not limited to them.

### Example 1

Evaluation of efficacy and safety of landiolol hydrochloride to the coronary angiography ability using MSCT:

In the imaging of MSCT, LightSpeed Ultra 16 (16-slices MSCT, manufactured by GE medical systems) was used.

As a Workstation, Advantage Workstation AW 4.2 (manufactured by GE Medical Systems) was used.

As an imaging analysis soft, CardIQ Analysis II manufactured by GE Medical Systems was used.

As contrast media, iomeprol 350 mgI/ml (brand name: Iomeron 350, manufactured by Eisai Co., Ltd., was used.

### (1) Administration of landiolol hydrochloride

To four angina pectoris patients, was intravenously administered landiolol hydrochloride swiftly over a period of 1 minute (0.063 mg/kg/minute), followed by a sustained intravenous administration over a period of 10 minutes (0.02 mg/kg/minute).

### (2) Coronary angiography by MSCT (administration of contrast media and method for imaging)

The contrast media were administered 10 minutes after confirming that the heart rate was slowed down. The imaging timing was determined by a test injection method using contrast media. As a result of swift administration of iomeprol (10 ml) and saline (20 ml) sequentially, it took approximately average of 16 seconds for iomeprol to reach heart. Afterwards, iomeprol (70 ml) and saline (20 ml) were swiftly administered sequentially and in time with the timing when iomeprol reached heart, MSCT imaging was started.

Radiographic demonstration of a coronary artery was done with (A) volume rendering (VR/three-dimensional image) method, (B) curved MPR (multi planar reformation) method and (C) vessel analysis (vessel automatic detection). Three-dimensional overall figures of coronary artery were grasped with (A), and then followed by evaluation of the lesion site with (B) and (C), optionally followed by addition of an orthogonal cross-section imaging of a coronary artery for further confirmation.

### Results:

To the above four angina pectosis patients, landiolol hydrochloride was administered. Table 1 shows the changes of the heart rate and the blood pressure of systolic blood pressure. The inhibition rate (%) was calculated by {(post-value / (pre-value) × 100-100}.

**Table 1**

| | Before administration (n=4) | After administration (n=4) | Inhibition rate (%) |
|---|---|---|---|
| Heart rate (/minute) | 61.0 | 54.8 | -10.2 |
| Systolic blood pressure (mmHg) | 173.3 | 161.8 | -6.6 |

As is apparent from table 1, by the administration of landiolol hydrochloride, the decrease of the heart rate was confirmed. As a result, the coronary angiography by MSCT was extremely sharp and it is concluded that the ability of coronary angiography was improved accompanying the effect of slowing down the heart rate.

As is apparent from table 1, it was confirmed that the heart rate was slowed down by administration of landiolol hydrochloride. As a result, the imaging of coronary angiography by MSCT was extremely sharp and the decrease of the heart rate rendered the higher resolution.

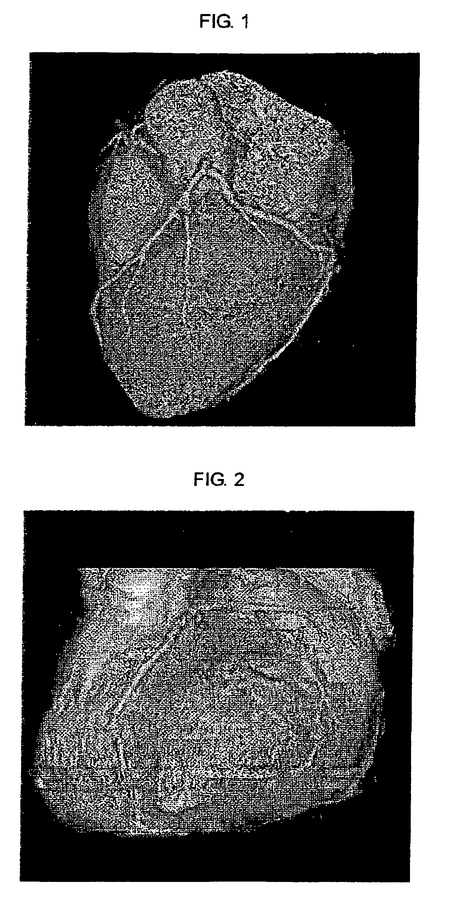

Fig. 1 shows one case of the angiography when landiolol hydrochloride was pre-administered. On the other hand, Fig. 2 shows one case of the angiography when landiolol hydrochrolide was not pre-administered.

To compare these figures, Fig. 1 shows up a sharp image of coronary arteries, whereas in Fig. 2 horizontal noises are standing out in the angiography, so it is obvious that the angiography of the coronary artery is not sharp. And since no excessive lowering of the blood pressure was found, the efficacy and safety of the present drug were recognized.

From these, landiolol hydrochloride is a useful agent for the purpose of gaining a sharp coronary angiography. Moreover, since the efficacy of the drug disappears in a short period, adverse effects such as staggering or qualm do not occur resulting from continuation of the decreased heart rate after performing the imaging, resulting in minimum burden to the patients on whom the imaging was performed.

### Formulation Example 1

### Preparation of a freeze-dried formulation comprising 50 mg of landiolol hydrochloride:

The following components were admixed by a conventional method, and the resulting solution was sterilized by a conventional method, placed 5 ml portions into vials and freeze-dried to give 10,000 vials each containing 50 mg of active ingredient.

| | |
|---|---|
| Landiolol hydrochloride | 500 g |
| D-Mannitol | 500 g |
| Sodium hydroxide | 420 mg |
| Distilled water | 6000 ml (total) |

### Formulation Example 2

### Preparation of a liquid formulation comprising 50 mg of landiolol hydrochloride:

The following components were admixed by a conventional method and the resulting solution was sterilized by a conventional method. The solution was filled in vials each 5 ml to give 10,000 vials of liquid solution each containing 50 mg of active ingredient.

| | |
|---|---|
| Landiolol hydrochloride | 500 g |
| D-Mannitol | 500 g |
| Sodium hydroxide | 420 mg |
| Distilled water | 6000 ml (total) |

### Industrial Applicability

A short-acting β-blocker is quick in expressing and disappearing the effect of the drug and it makes it possible to control the heart rate for a short period, which is necessary for coronary angiography represented by MSCT. Therefore, it can shorten the observation time considerably; waiting time from administration to expressing of the drug efficacy, from inspection to disappearing of the drug efficacy after diagnosis, compared with existing oral β-blocker. It can reduce the time burden of a patient in MSCT inspection. Also, for medical institution, they do not have to force the patient to take a medicine several hours before the diagnosis. The diagnosis is done in a short period when the patient comes to the clinic.

Unlike oral β-blockers, it is possible to control the heart rate by adjusting the dose. In particular, those patients in out-patient clinic, they can go back to their daily life soon after checkup without lingering thin pulse.

A short-acting β-blocker is excellent in controlability and therefore it is useful as a diagnostic auxiliary in diagnostic imaging.

## Claims

1. A compound which is landiolol hydrochloride for use in slowing the heart rate of a patient during diagnostic imaging using multi-slice helical computed tomography (MSCT), wherein the compound is administered in a swift intravenous administration of the compound at 0.063 to 0.125 mg/kg/minute for 30 seconds to 3 minutes.

2. The compound according to claim 1 for use according to claim 1, wherein the landiolol hydrochloride is provided to the patient in said swift intravenous administration additionally followed by administration of the compound at 0.01 to 0.08 mg/kg/minute for 5 to 20 minutes.

3. A compound which is landiolol hydrochloride for use in slowing the heart rate of a patient during diagnostic imaging using multi-slice helical computed tomography (MSCT), wherein the landiolol hydrochloride is provided to the patient in a swift intravenous administration of from 0.063 to 0.125 mg/kg/minute of from 30 seconds to 3 minutes followed by a sustained intravenous administration of from 0.01 to 0.08 mg/kg/minute of from 5 to 20 minutes.

4. The compound according to claim 1 for use according to claim 1, 2 or 3, wherein the diagnostic imaging is diagnostic imaging for heart diseases.

5. The compound according to claim 1 for use according to any preceding claim, wherein a contrast medium is also administered.

6. The compound according to claim 1 for use according to claim 5, wherein the contrast medium is chosen from an iodinated contrast medium, a borated contrast medium, a xenon contrast medium, a barium contrast medium, a ferrous contrast medium and a gadolinium contrast medium.

## Patentansprüche

1. Verbindung, welche Landiolol-Hydrochlorid ist, zur Verwendung beim Verlangsamen der Herzfrequenz eines Patienten während einer diagnostischen Bildgebung unter Verwendung einer Mehrzeilen-Spiral-Computertomographie (MSCT), wobei die Verbindung in einer schnellen intravenösen Verabreichung der Verbindung mit 0,063 bis 0,125 mg/kg/Minute während 30 Sekunden bis 3 Minuten verabreicht wird.

2. Verbindung nach Anspruch 1 zur Verwendung nach Anspruch 1, wobei das Landiolol-Hydrochlorid dem Patienten in der schnellen intravenösen Verabreichung gegeben wird, zusätzlich gefolgt von einer Verabreichung der Verbindung mit 0,01 bis 0,08 mg/kg/Minute während 5 bis 20 Minuten.

3. Verbindung, welche Landiolol-Hydrochlorid ist, zur Verwendung beim Verlangsamen der Herzfrequenz eines Patienten während einer diagnostischen Bildgebung unter Verwendung einer Mehrzeilen-Spiral-Computertomographie (MSCT), wobei das Landiolol-Hydrochlorid dem Patienten in einer schnellen intravenösen Verabreichung von 0,063 bis 0,125 mg/kg/Minute während 30 Sekunden bis 3 Minuten gegeben wird, gefolgt von einer verzögerten intravenösen Verabreichung von 0,01 bis 0,08 mg/kg/Minute während 5 bis 20 Minuten.

4. Verbindung nach Anspruch 1 zur Verwendung nach Anspruch 1, 2 oder 3, wobei die diagnostische Bildgebung eine diagnostische Bildgebung für Herzkrankheiten ist.

5. Verbindung nach Anspruch 1 zur Verwendung nach einem der vorangehenden Ansprüche, wobei auch ein Kontrastmittel verabreicht wird.

6. Verbindung nach Anspruch 1 zur Verwendung nach Anspruch 5, wobei das Kontrastmittel ausgewählt ist aus einem jodierten Kontrastmittel, einem boratierten Kontrastmittel, einem Xenon-Kontrastmittel, einem Barium-Kontrastmittel, einem eisenhaltigen Kontrastmittel und einem Gadolinium-Kontrastmittel.

## Revendications

1. Composé qui est le chlorhydrate de landiolol utilisable pour ralentir le rythme cardiaque d'un patient lors d'une procédure d'imagerie diagnostique par tomodensitométrie (TDM) multicoupe, dans lequel le composé est administré par voie intraveineuse rapide à raison de 0,063 à 0,125 mg/kg/minute de composé pendant 30 secondes à 3 minutes.

2. Composé selon la revendication 1 utilisable selon la revendication 1, dans lequel le chlorhydrate de landiolol est administré au patient par ladite voie intraveineuse rapide, cette administration étant, en plus, suivie de l'administration du composé à raison de 0,01 à 0,08 mg/kg/minute pendant 5 à 20 minutes.

3. Composé qui est le chlorhydrate de landiolol utilisable pour ralentir le rythme cardiaque d'un patient lors d'une procédure d'imagerie diagnostique par tomodensitométrie (TDM) multicoupe, dans lequel le chlorhydrate de landiolol est administré au patient par voie intraveineuse rapide à raison de 0,063 à 0,125 mg/kg/minute pendant 30 secondes à 3 minutes, cette administration étant suivie d'une administration par voie intraveineuse soutenue à raison de 0,01 à 0,08 mg/kg/minute de composé pendant 5 à 20 minutes.

4. Composé selon la revendication 1 utilisable selon les revendications 1, 2 ou 3, dans lequel la procédure d'imagerie diagnostique est une procédure d'imagerie diagnostique pour les maladies du coeur.

5. Composé selon la revendication 1 utilisable selon l'une quelconque des revendications précédentes, dans lequel un milieu de contraste est également administré.

6. Composé selon la revendication 1 utilisable selon la revendication 5, dans lequel le milieu de contraste est choisi parmi un milieu de contraste à l'iode, un milieu de contraste au bore, un milieu de contraste au xénon, un milieu de contraste au baryum, un milieu de contraste ferreux et un milieu de contraste au gadolinium.
